# EUROPEAN PATENT SPECIFICATION

(11) **EP 4 636 140 B1**
(45) Date of publication and mention of the grant of the patent: **29.04.2026**
(21) Application number: 25163503.3
(22) Date of filing: 13.03.2025
(51) Int. Cl.: D01D 13/02, G01N 33/36, G01J 5/00, G01K 11/00, G01N 25/72

(54) **YARN SPINNING SYSTEM**
GARNSPINNSYSTEM
SYSTÈME DE FILAGE DE FIL

(30) Priority: 12.04.2024 JP 2024064637
(43) Date of publication of application: 22.10.2025
(73) Proprietor: TMT Machinery, Inc., Osaka-shi, Osaka 541-0041 (JP)
(72) Inventor: MATSUDA, Ryo, Kyoto-shi, Kyoto, 612-8686 (JP); YOSHIDA, Kazuto, Kyoto-shi, Kyoto, 612-8686 (JP)
(74) Representative: Hoffmann Eitle

(56) References cited:
- EP-A2- 1 148 323
- CN-A- 113 913 950
- DE-B4- 102022 132 168
- JP-A- 2023 084 690

## Description

### BACKGROUND OF THE INVENTION

The present invention relates to a yarn spinning system configured to produce a spun yarn.

Patent Literature 1 (Japanese Laid-Open Patent Publication No. 2023-53671) discloses a yarn spinning system including a spinning apparatus configured to spin out a yarn. A spinning pack is attachable to and detachable from the spinning apparatus. The spinning apparatus is configured to spin out hot molten polymer, which is supplied to the attached spinning pack, through nozzles formed at a spinneret of the spinning pack. Such a yarn spinning system needs regular maintenance such as cleaning of the spinneret, etc. Further relevant information can also be found in documents EP 1 148 323 A2, JP 2023 084690 A, CN 113 913 950 A and DE 10 2022 132168.

### SUMMARY OF THE INVENTION

The quality of a yarn produced in the above-described yarn spinning system varies depending on the temperature of the spinneret. For example, when yarn production is restarted after the maintenance of the spinneret, (i) the temperature of the spinneret and (ii) the quality of the yarn spun out from the spinning apparatus are low. It is therefore necessary to waste the yarn spun out from the spinning apparatus, until the temperature of the spinneret is sufficiently raised. The time during which the yarn is wasted is set based on experience, and whether this time is appropriate is unclear. In this regard, a thermocouple may be attached to the spinneret to measure the temperature of the spinneret. However, the nozzles configured to discharge the molten polymer are gathered at the spinneret, and it is difficult to attach the thermocouple to a position with which the polymer does not make contact. To solve this, an infrared light sensor may be provided below the spinneret to directly measure the temperature of the spinneret in a contactless manner. In this case, the infrared light sensor needs to be provided at a position on which a beam of infrared light radiated from the spinneret is incident. Therefore, the position of the infrared light sensor is in the vicinity of a yarn path of the yarn spun out from the spinneret. As a result, the infrared light sensor is disadvantageously damaged by the falling hot molten polymer or contaminated by the falling yarn dust so that measurement of temperature is impossible.

An object of the present invention is to provide a yarn spinning system in which the temperature of a spinneret is easily graspable in a contactless manner.

According to a first aspect of the invention, a yarn spinning system comprises: at least one spinning apparatus including at least one spinneret configured to spin out a yarn; at least one reflector which is provided at a position on which at least one beam of infrared light radiated from the at least one spinneret is incident, and which is configured to reflect the at least one incident beam of the infrared light; and an infrared light sensor which is able to measure the temperature of the at least one spinneret by receiving the at least one beam of the infrared light reflected by the at least one reflector, and the at least one reflector being opposite to the infrared light sensor over a yarn path of the yarn spun out from the at least one spinneret.

According to this aspect, the at least one reflector is provided relatively in the vicinity of the yarn path of the yarn so that the at least one beam of the infrared light radiated from the at least one spinneret is incident. Meanwhile, the infrared light sensor may be differently arranged as long as it is provided at a position where the at least one beam of the infrared light reflected by the at least one reflector is received. That is, the infrared light sensor can be provided at a position which is relatively far from the yarn path of the yarn on the side opposite to the at least one reflector over the yarn path of the yarn. With this arrangement, the infrared light sensor is less likely to be damaged by hot molten polymer or to be contaminated by yarn dust. As a result, a problem in which the measurement of temperature is impossible is also less likely to occur. This makes it possible to easily grasp the temperature of the at least one spinneret in a contactless manner.

According to a second aspect of the invention, the yarn spinning system of the first aspect is arranged such that the infrared light sensor is able to measure the temperature distribution of the at least one spinneret.

According to this aspect, the existence of unevenness in temperature of the at least one spinneret, etc. is grasped by the measurement of temperature distribution of the at least one spinneret by the infrared light sensor. It is therefore possible to reliably suppress the production of a low-quality yarn.

According to a third aspect of the invention, the yarn spinning system of the first or second aspect is arranged such that the at least one reflector is able to reflect beams of the infrared light radiated from spinnerets.

According to this aspect, the at least one reflector is configured to reflect the beams of the infrared light radiated from the spinnerets. With this arrangement, the infrared light sensor is able to measure the temperature of each of the spinnerets by receiving each of the beams of the infrared light reflected by the at least one reflector. It is therefore possible to easily grasp how uneven the temperature is between the spinnerets.

According to a fourth aspect of the invention, the yarn spinning system of any one of the first to third aspects is arranged such that the infrared light sensor is able to receive the beams of the infrared light at once which are radiated from the spinnerets and which are reflected by the at least one reflector.

According to this aspect, the infrared light sensor is able to measure the temperature of the spinnerets at once. This shortens the time of measurement.

According to a fifth aspect of the invention, the yarn spinning system of any one of the first to fourth aspects further comprises a movable moving body. In this yarn spinning system, the infrared light sensor is provided at the moving body, and as the moving body moves, the infrared light sensor is able to receive the beams of the infrared light which are radiated from the spinnerets and which are reflected by the at least one reflector.

According to this aspect, the infrared light sensor is able to measure the temperature of the spinnerets as the moving body moves.

According to a sixth aspect of the invention, the yarn spinning system of the fifth aspect comprises spinning apparatuses aligned in one direction. In this yarn spinning system, the moving body is movable across the spinning apparatuses, and as the moving body moves, the infrared light sensor is able to measure the temperature of each of the spinnerets of the spinning apparatuses.

According to this aspect, as the moving body moves, the temperature of the spinnerets of the spinning apparatuses can be measured by the infrared light sensor provided at the moving body. It is therefore unnecessary to provide the infrared light sensor for each of the spinning apparatuses when the spinning apparatuses are aligned in the one direction.

According to a seventh aspect of the invention, the yarn spinning system of any one of the first to sixth aspects further comprises a cooler which is provided below each of the spinning apparatuses and which is configured to cool the yarn spun out from the each of the spinnerets. In this yarn spinning system, the at least one reflector is provided below the cooler.

When the cooler is provided below the each of the spinning apparatuses, the infrared light sensor needs to be provided further in the vicinity of the yarn path of the yarn so that the temperature of the each of the spinnerets is directly measured by the infrared light sensor. Because of this, the infrared light sensor is more likely to be damaged by the falling hot molten polymer or to be contaminated by the falling yarn dust. As a result, a problem in which the measurement of temperature is impossible is also more likely to occur. Therefore, it is further effective to adopt this aspect in which (i) the at least one reflector which is more resistant to damage and contamination than the infrared light sensor is provided in the vicinity of the yarn path of the yarn, (ii) the beams of the infrared light radiated from the spinnerets are reflected by the at least one reflector, and (iii) the reflected beams of the infrared light are received by the infrared light sensor.

According to an eighth aspect of the invention, the yarn spinning system of any one of the first to seventh aspects further comprises an oil applicator which is provided below the each of the spinning apparatuses and which includes an oil supply guide configured to apply oil to the yarn spun out from the each of the spinnerets. In this yarn spinning system, the at least one reflector is attached to the oil applicator.

It is relatively specious around the oil applicator. This facilitates the attachment of the at least one reflector.

According to a ninth aspect of the invention, the yarn spinning system of the eighth aspect is arranged such that the oil applicator further includes a cover which is provided above the oil supply guide and which is movable between a position where the cover does not cover the oil supply guide and a position where the cover covers the oil supply guide, and the cover functions as the at least one reflector.

According to this aspect, the number of members is reduced by using the cover of the oil applicator as the at least one reflector.

According to a tenth aspect of the invention, the yarn spinning system of any one of the first to ninth aspects is arranged such that the at least one reflector is able to move up and down, and an angle adjusting mechanism is provided to adjust a reflection angle of each of the beams of the infrared light at the at least one reflector.

According to this aspect, even when the position of the each of the one or more reflectors is changed in the up-down direction, the angle adjusting mechanism adjusts the reflection angle of each of the beams of the infrared light at the at least one reflector so that the beams of the infrared light radiated from the spinnerets are reflected toward the infrared light sensor by the at least one reflector.

According to an eleventh aspect of the invention, the yarn spinning system of the tenth aspect is arranged such that the infrared light sensor is able to move up and down.

According to this aspect, the infrared light sensor is able to move up and down as the at least one reflector move up and down. The infrared light sensor is therefore able to receive the beams of the infrared light reflected by the at least one reflector, at a suitable position.

### BRIEF DESCRIPTION OF THE DRAWINGS

FIG. 1 is a schematic diagram of a yarn spinning system of an embodiment.
FIG. 2 is a front view of a spinning apparatus and its surroundings.
FIG. 3 is a bottom view of a cooler.
FIG. 4 is a side view of the spinning apparatus and its surroundings in package production.
FIG. 5 is a side view of the spinning apparatus and its surroundings in yarn threading.
FIG. 6 is a side view of an oil applicator and its surroundings.
FIG. 7 is a grayscale image taken by a thermal camera.

### DESCRIPTION OF THE PREFERRED EMBODIMENTS

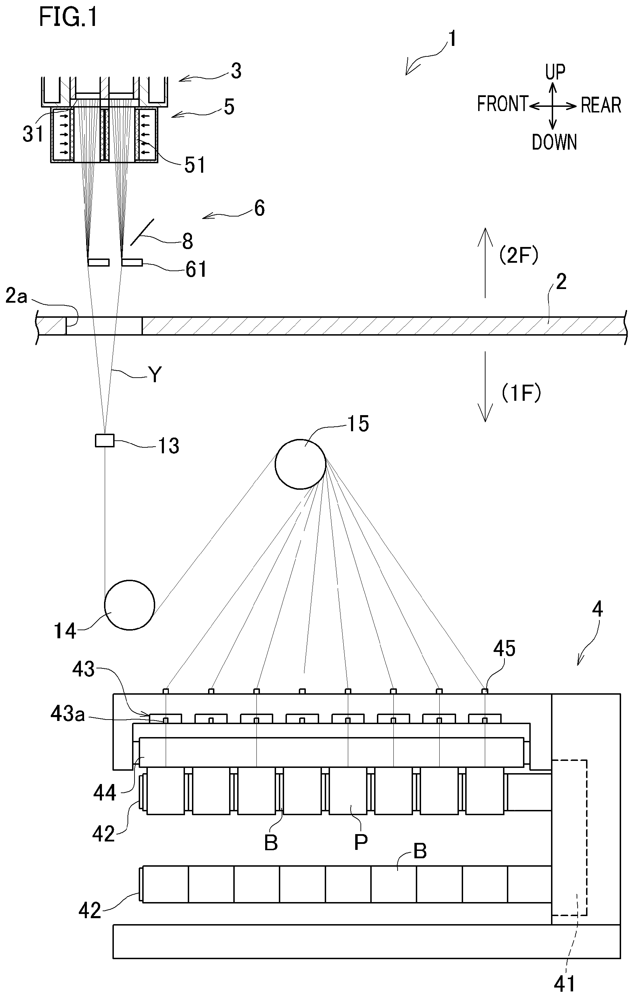

The following will describe a yarn spinning system 1 of a preferred embodiment of the present invention, with reference to FIG. 1 to FIG. 6. Hereinafter, the up-down direction on the sheet of FIG. 1 will be used as the up-down direction of the yarn spinning system 1 (the vertical direction in which the gravity acts). The left-right direction on the sheet of FIG. 1 will be used as the front-rear direction of the yarn spinning system 1. The direction perpendicular to the sheet of FIG. 1 will be used as the left-right direction of the yarn spinning system 1. The front-rear direction and the left-right direction extend along a horizontal direction. The front-rear direction is orthogonal to the left-right direction.

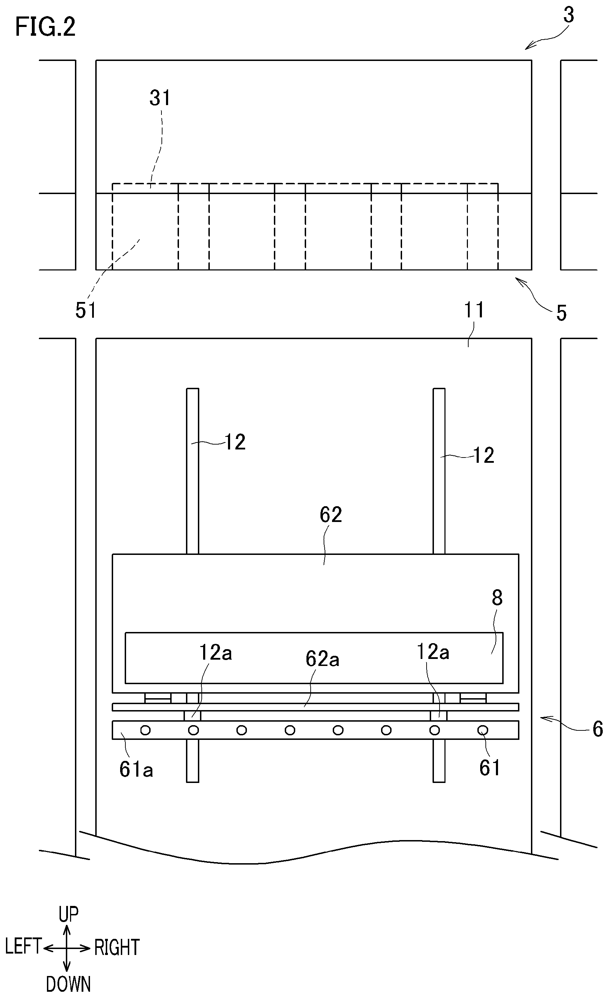

As shown in FIG. 1, the yarn spinning system 1 is partitioned into a first floor (lower floor) and a second floor (upper floor) by a partition floor 2. The partition floor 2 is provided with an opening 2a which causes the first floor to communicate with the second floor. The yarn spinning system 1 mainly includes a spinning apparatus 3 provided on the second floor and a winding device 4 provided on the first floor. The yarn spinning system 1 is structured so that yarns Y spun-out from each spinning apparatus 3 are brought down to the first floor through the opening 2a and wound by each winding device 4, so as to produce packages P. These yarns Y are made of synthetic resin. The yarn spinning system 1 is structured so that (i) spinning apparatuses 3 are aligned in the left-right direction and (ii) winding devices 4 are aligned in the left-right direction. FIG. 2 illustrates only one spinning apparatus 3.

Each spinning apparatus 3 includes spinnerets 31. In the present embodiment, the spinnerets 31 are staggered to form two lines extending along the left-right direction. The arrangement manner of the spinnerets 31 is not limited to a staggered manner. The spinnerets 31 may be aligned to form a single line extending along the left-right direction.

As hot molten polymer is pushed out from nozzles (not illustrated) of the spinnerets 31, the yarns Y are spun out from each spinning apparatus 3. To be exact, thin yarns which are immediately after being spun out from each spinneret 31 are called filaments. The filaments spun out from each spinneret 31 are combined into a single yarn Y by a later-described oil supply guide 61. In the present embodiment, eight yarns Y are spun out from the spinning apparatus 3 including eight spinnerets 31, and eight packages P are formed by the winding device 4. However, the number of the yarns Y, the spinnerets 31, and the packages P is suitably changeable.

As shown in FIG. 1, a cooler 5 configured to cool the yarns Y spun out from the spinnerets 31 and an oil applicator 6 including oil supply guides 61 configured to apply oil to the yarns Y spun out from the spinnerets 31 are provided on the second floor in addition to the spinning apparatus **3.**

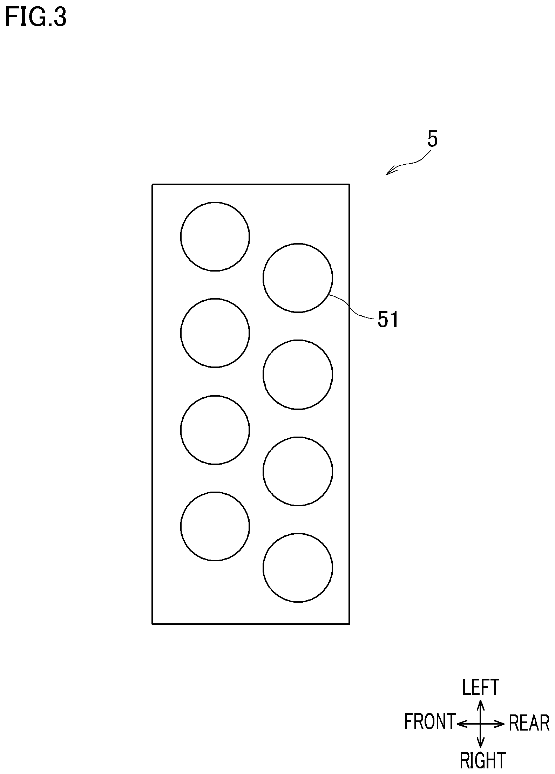

The cooler 5 is provided below the spinning apparatus **3.** As shown in **FIG. 2****,** the cooler 5 includes cooling cylinders 51 aligned to correspond to the spinnerets 31. Each cooling cylinder 51 is provided immediately below a corresponding spinneret 31. Similarly to the spinnerets 31, the cooling cylinders 51 are also staggered to form two lines extending along the left-right direction. To be more specific, as shown in FIG. 3, each of these two lines is formed of four cooling cylinders 51 aligned in the left-right direction. In this regard, the positions of the cooling cylinders 51 are different between the lines in the left-right direction. When the yarns Y spun out from the spinnerets 31 pass through the cooling cylinders 51, the yarns Y are cooled and solidified by gas supplied to the cooling cylinders 51.

The cooler 5 is able to move up and down by means of a movement mechanism (not illustrated). The position of the cooler 5 in production of the packages P is a position where the cooler 5 is in contact with a lower end of the spinning apparatus 3 (a position shown in FIG. 1). In maintenance such as cleaning of the spinnerets 31, etc., the cooler 5 is moved downward so that a working space is formed between the spinning apparatus 3 and the cooler 5.

The oil applicator 6 is provided below the cooler 5. As shown in FIG. 2, the oil applicator 6 includes the oil supply guides 61 aligned to correspond to the spinnerets 31. Similarly to the spinnerets 31, the oil supply guides 61 are also staggered to form two lines extending along the left-right direction. The oil supply guides 61 are attached to a bracket 61a extending along the left-right direction. The oil supply guides 61 are configured to apply oil to the yarns Y having been solidified by the cooler 5.

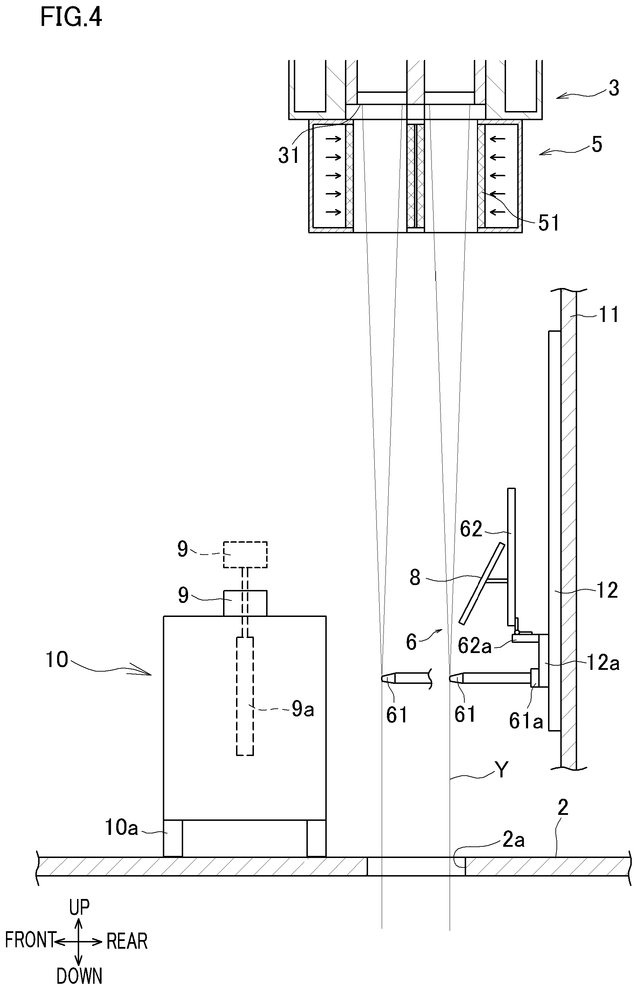

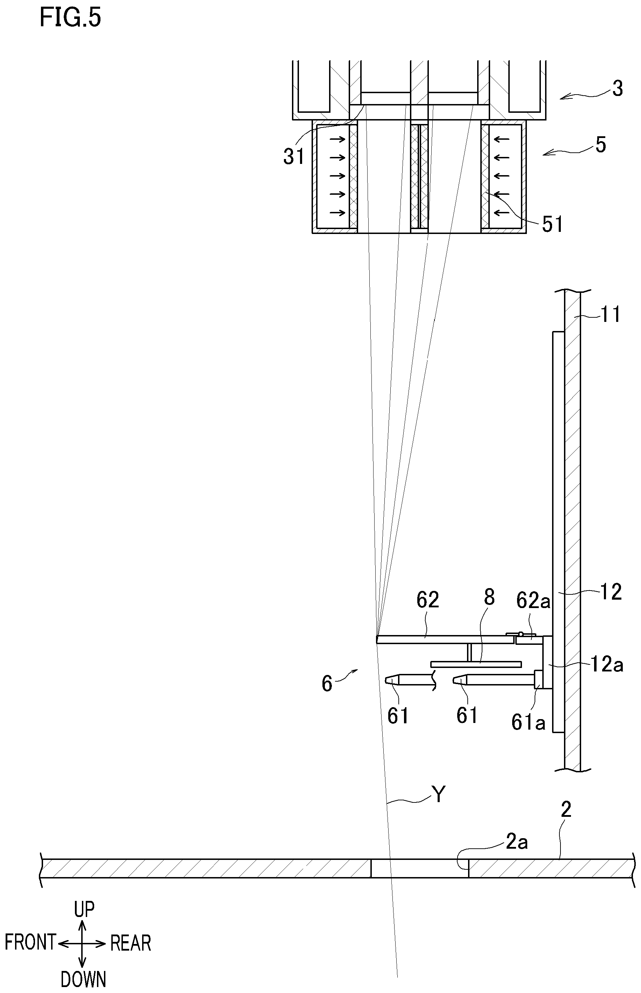

As shown in FIG. 2 and FIG. 4, the oil applicator 6 further includes a cover 62 provided above the oil supply guides 61. The cover 62 is a plate-shaped member extending in the left-right direction. The cover 62 is swingable about an axis extending along the left-right direction, by means of a hinge. The cover 62 is able to swing and move between a retracted position (see FIG. 4) where the cover 62 does not cover the oil supply guides 61 and a covering position (see FIG. 5) where the cover 62 covers the oil supply guides 61. A thickness direction of the cover 62 at the retracted position extends substantially in the front-rear direction. The cover 62 at the retracted position is positioned behind the cooling cylinders 51. The thickness direction of the cover 62 at the covering position extends substantially in the up-down direction.

The cover 62 is provided for preventing the yarns Y from being entangled with the oil supply guides 61 until yarn threading to the oil supply guides 61 is performed. This yarn threading is performed when the production of the packages P is started in the yarn spinning system 1. In the yarn threading, to begin with, an operator on the second floor brings the yarns Y spun out from the spinning apparatus 3 down to the first floor through the opening 2a, and then an operator on the first floor sucks and holds the brought-down yarns Y by means of a suction gun. Subsequently, after the yarns Y are sucked and held by the suction gun and are tensioned, the operator on the second floor threads the yarns Y to the respective oil supply guides 61. Meanwhile, the operator on the first floor threads the yarns Y which are sucked and held by the suction gun to sections provided on the first floor.

When the above-described yarn threading is performed, the cover 62 is positioned at the covering position (see FIG. 5) so that the yarns Y are not entangled with the oil supply guides 61 until the yarn threading to the oil supply guides 61 is performed. When the packages P are produced, the cover 62 is positioned at the retracted position (see FIG. 4) so as not to interfere with the yarns Y.

The distance from the spinnerets 31 to the oil supply guides 61 needs to be changed depending on the type of the yarns Y to be produced. Therefore, the oil applicator 6 is able to move up and down. The following will describe the movement mechanism of the oil applicator 6.

As shown in FIG. 2 and FIG. 4, a wall member 11 is provided below the cooler 5. The thickness direction of the wall member 11 extends along the front-rear direction. The width (length in the left-right direction) of the wall member 11 is substantially the same as that of the cooler 5. A pair of left and right rails 12 are attached to a front surface of the wall member 11. Each rail 12 extends along the up-down direction. A slider 12a which is able to slide up and down along each rail 12 is fitted to each rail 12. Each slider 12a may be configured to move up and down by means of a driving source such as a motor, etc. Alternatively, an operation portion such as a handle, etc. operated by the operator may be provided so that each slider 12a moves up and down manually as the operator operates the operation portion.

The bracket 61a to which the oil supply guides 61 are attached is supported by two sliders 12a. The cover 62 is supported by the two sliders 12a via an attaching member 62a. With this arrangement, as the sliders 12a move up and down, the oil supply guides 61 and the cover 62 which form the oil applicator 6 also move up and down.

As shown in FIG. 2 and FIG. 4, a reflector 8 is attached to the cover 62. The reflector 8 is provided at a position on which beams of infrared light radiated from the spinnerets 31 are incident. In the present embodiment, the single reflector 8 is attached to the cover 62. The single reflector 8 is provided at a position on which beams of infrared light radiated from all the spinnerets 31 included in each spinning apparatus 3 are incident. That is, beams of infrared light radiated from the eight spinnerets 31 are incident on the single reflector 8.

The reflector 8 is able to reflect the incident beams of infrared light. As described later, a thermal camera 9 is configured to receive the beams of infrared light reflected by the reflector 8. The reflector 8 is made of, e.g., metal. Among the metal, it is preferable to use aluminum, silver, copper, etc. with a relatively high reflectivity of beams of infrared light which has a wavelength of 8 µm to 14 µm which is received by the thermal camera 9. Alternatively, iron or stainless may be used for the reflector 8 in consideration of the availability of a material in addition to the refractivity of beams of infrared light. The material of the reflector 8 is not limited to the above-described materials.

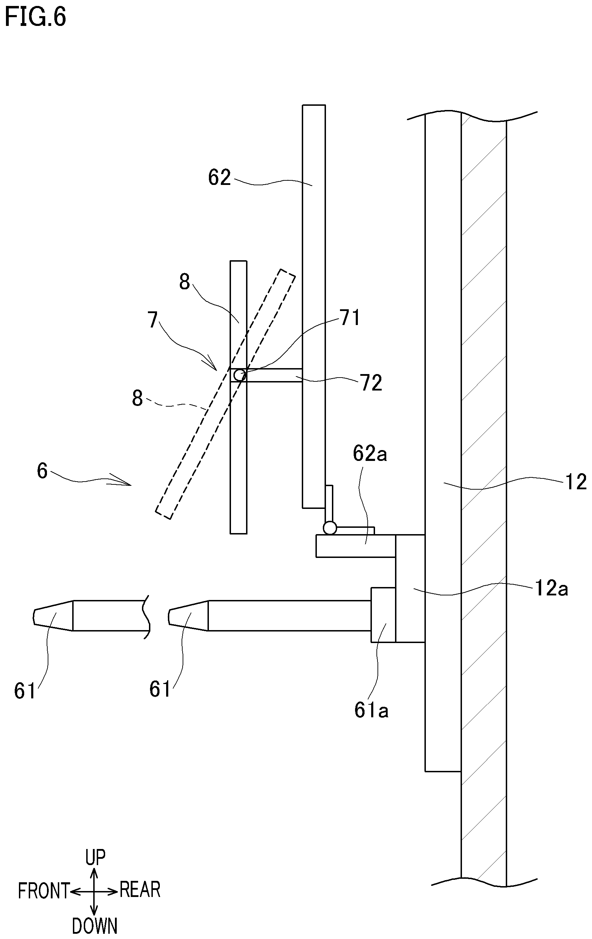

As shown in FIG. 4, the reflector 8 is attached to a front surface of the cover 62 at the retracted position. As shown in FIG. 6, the reflector 8 is able to adjust a reflection angle of each beam of infrared light at the reflector 8 by means of an angle adjusting mechanism 7. The angle adjusting mechanism 7 includes, e.g., a supporting shaft 71 extending along the left-right direction and an attaching member 72 attaching the supporting shaft 71 to the cover 62. The reflector 8 is supported by the supporting shaft 71 to be swingable about the supporting shaft 71. As the reflector 8 swings about the supporting shaft 71, the orientation of surfaces of the reflector 8 changes so that the reflection angle of each beam of the infrared light at the reflector 8 also changes.

Referring back to FIG. 1, a separation guide 13 is provided immediately below the opening 2a of the partition floor 2. The separation guide 13 is a comb teeth guide provided for regulating the intervals of the yarns Y, which are brought down from the second floor through the opening 2a, to predetermined intervals. Godet rollers 14 and 15 are provided downstream of the separation guide 13 in a yarn running direction. Each of the godet rollers 14 and 15 is rotationally driven by an unillustrated motor. The yarns Y are sent to the winding device 4 by the godet rollers 14 and 15.

The winding device 4 includes a turret 41, two bobbin holders 42, a traverse device 43, a contact roller 44, etc. The two bobbin holders 42 are rotatably supported by the turret 41. As the turret 41 rotates, the positions of the two bobbin holders 42 are reversed upside down. To each bobbin holder 42, bobbins B are attached. The traverse device 43 includes traverse guides 43a corresponding the respective bobbins B attached to each bobbin holder 42. As each traverse guide 43a reciprocates, a yarn Y is wound onto a bobbin B while being traversed about a corresponding fulcrum guide 45, with the result that a package P is formed. The contact roller 44 is configured to make contact with packages P formed on the upper bobbin holder 42 so as to apply contact pressure to each package P.

As shown in FIG. 4, the yarn spinning system 1 includes a running body 10 configured to run on the partition floor 2 of the second floor. In the present embodiment, the running body 10 is configured to run with a tire 10a. The running body 10 is movable in any direction on the partition floor 2. That is, the running body 10 is movable in the left-right direction (the arrangement direction of spinning apparatuses 3). The running body 10 is movable across the spinning apparatuses 3 by running along the left-right direction. The running body 10 may be configured to run autonomously or may be configured to run on a route set in advance. Alternatively, the running body 10 may be operated by the operator.

The thermal camera 9 is attached to the running body 10. The thermal camera 9 is able to move up and down by means of a movement mechanism 9a such as an air cylinder, etc. The running body 10 is configured to run on the side opposite to the wall member 11 over the opening 2a of the partition floor 2 in the front-rear direction. That is, the reflector 8 attached to the cover 62 supported by the wall member 11 is opposite to the thermal camera 9 attached to the running body 10 over yarn paths of the yarns Y spun out from the spinnerets 31.

When the running body 10 is positioned in front of the wall member 11, the thermal camera 9 receives a beam of infrared light which is radiated from each spinneret 31 and which is reflected by the reflector 8. The thermal camera 9 then measures the temperature of each spinneret 31 based on the amount of energy of the received beam of infrared light. The thermal camera 9 is able to measure the temperature distribution of each spinneret 31 by receiving beams of infrared light radiated from parts of each spinneret 31 via the reflector 8. In the present embodiment, the thermal camera 9 is able to receive beams of infrared light at once which are radiated from spinnerets 31 and which are reflected by the reflector 8. That is, for example, the thermal camera 9 is configured to receive beams of infrared light at once which are respectively radiated from two spinnerets 31 and which are reflected by the reflector 8.

As described above, in the present embodiment, the reflector 8 is provided at a position on which beams of infrared light radiated from all the spinnerets 31 are incident. With this arrangement, as the running body 10 moves, the thermal camera 9 receives beams of infrared light which are radiated from all the spinnerets 31 and which are reflected by the reflector 8. This allows the thermal camera 9 to measure the temperature distribution of each of all the spinnerets 31. As the running body 10 moves across the spinning apparatuses 3, the thermal camera 9 measures the temperature distribution of each of spinnerets 31 of the spinning apparatuses 3 aligned in the left-right direction.

For example, an image taken by the thermal camera 9 may be displayed on a display which is integrally formed with the thermal camera 9. Alternatively, an image may be sent to a controller of the yarn spinning system 1 via wireless communication, etc. so that the image is displayed on a display connected to the controller. Alternatively, an image may be sent to a mobile terminal such as a tablet terminal, etc.

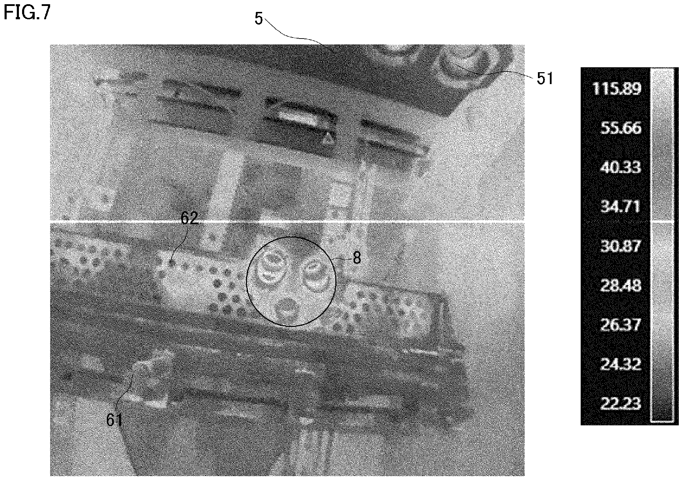

FIG. 7 shows a grayscale image taken by the thermal camera 9. In this image, temperature distribution is indicated by shades of gray. In the image shown in FIG. 7, the temperature distribution of spinnerets 31 is shown at the reflector 8.

### (Relationship Between Layout of Reflector 8 and Measured Temperature)

Investigation was performed for the relationship between the layout of the reflector 8 and the measured temperature of each spinneret 31. A thermal imager (testo 882) produced by Testo SE & Co. KGaA was used for the measurement of temperature. The reflectivity was set at 0.50. The measurement of temperature was performed when the yarns Y ware not spun out from the spinnerets 31. Firstly, when the temperature of each spinneret 31 was directly measured from a position immediately below the spinneret 31 without using the reflector 8, the temperature of the spinneret 31 was 319 °C.

Secondly, the cooler 5 was provided at a position separated from the lower end of the spinning apparatus 3 (provided at a position in the maintenance), and the reflector 8 was provided immediately below the spinning apparatus 3. In this case, when the temperature of the spinneret 31 was measured via the reflector 8, the temperature of the spinneret 31 was 310 °C. That is, the measured temperature of the spinneret 31 in this case is decreased from that (319 °C) of the spinneret 31 in the case where it is directly measured, by approximately 3 %. In this regard, the distance between the spinneret 31 and the reflector 8 is within 300 mm.

Thirdly, the cooler 5 was provided at a position in contact with the lower end of the spinning apparatus 3 (a position in package production), and the reflector 8 was provided immediately below the cooler 5. In this case, when the temperature of the spinneret 31 was measured via the reflector 8, the temperature of the spinneret 31 was 295 °C. That is, the measured temperature of the spinneret 31 in this case is decreased from that (319 °C) of the spinneret 31 in the case where it is directly measured, by approximately 8 %. In this regard, the distance between the spinneret 31 and the reflector 8 is approximately 800 mm.

As such, the longer the distance between the spinneret 31 and the reflector 8 is, the lower the measured temperature of the spinneret 31 with the reflector 8 is from the temperature of the spinneret 31 directly measured. It is therefore possible to estimate how much the measured temperature of the spinneret 31 in the case where it is measured via the reflector 8 is decreased from the measured temperature of the spinneret 31 in the case where it is directly measured, based on the distance between the spinneret 31 and the reflector 8 to some extent.

### (Characteristics of Embodiment)

As described above, the yarn spinning system 1 of the present embodiment includes: the spinning apparatus 3 including the spinnerets 31 configured to spin out the yarns Y; the reflector 8 which is provided at a position on which beams of infrared light radiated from the spinnerets 31 are incident, and which is configured to reflect the incident beams of infrared light; and the thermal camera 9 which is able to measure the temperature of each spinneret 31 by receiving the beams of infrared light reflected by the reflector 8. The reflector 8 is opposite to the thermal camera 9 over the yarn paths of the yarns Y spun out from the spinnerets 31.

In the present embodiment, the reflector 8 is provided relatively in the vicinity of the yarn paths of the yarns Y so that the beams of infrared light radiated from the spinnerets 31 are incident. Meanwhile, the thermal camera 9 may be differently arranged as long as it is provided at a position where the beams of infrared light reflected by the reflector 8 are received. That is, the thermal camera 9 can be provided at a position which is relatively far from the yarn paths of the yarns Y on the side opposite to the reflector 8 over the yarn paths of the yarns Y. With this arrangement, the thermal camera 9 is less likely to be damaged by the hot molten polymer or to be contaminated by yarn dust. As a result, a problem in which the measurement of temperature is impossible is also less likely to occur. This makes it possible to easily grasp the temperature of each spinneret 31 in a contactless manner. For example, when the yarn production is restarted after the maintenance of the spinnerets 31, whether the temperature of the spinnerets 31 is raised to a temperature suitable for the yarn production is checked by grasping the temperature of the spinnerets 31. During the yarn production, when the temperature of the spinnerets 31 goes out of the temperature suitable for the yarn production, control, etc. is performed so that the temperature of the spinnerets 31 goes back to the suitable temperature.

In the yarn spinning system 1 of the present embodiment, the thermal camera 9 is able to measure the temperature distribution of each spinneret 31. In the present embodiment, the existence of unevenness in temperature of each spinneret 31 is grasped by the measurement of temperature distribution of each spinneret 31 by the thermal camera 9. It is therefore possible to reliably suppress the production of a low-quality yarn Y.

In the yarn spinning system 1 of the present embodiment, the reflector 8 is able to reflect beams of infrared light radiated from the spinnerets 31. In the present embodiment, the thermal camera 9 is able to measure the temperature of each of the spinnerets 31 by receiving the beams of infrared light reflected by the reflector 8. It is therefore possible to grasp how uneven the temperature is between the spinnerets 31.

In the yarn spinning system 1 of the present embodiment, the thermal camera 9 is able to receive the beams of infrared light at once which are radiated from the spinnerets 31 and which are reflected by the reflector 8. In the present embodiment, the thermal camera 9 is able to measure the temperature of the spinnerets 31 at once. This shortens the time of measurement.

The yarn spinning system 1 of the present embodiment further includes the movable running body 10, and the thermal camera 9 is provided at the running body 10. The thermal camera 9 is able to receive beams of infrared light which are radiated from the spinnerets 31 and which are reflected by the reflector 8, as the running body 10 moves. In the present embodiment, the thermal camera 9 is able to measure the temperature of the spinnerets 31 as the running body 10 moves.

The yarn spinning system 1 of the present embodiment includes the spinning apparatuses 3 aligned in the left-right direction, and the running body 10 is able to move across the spinning apparatuses 3. The thermal camera 9 is able to measure the temperature of the spinnerets 31 of the spinning apparatuses 3 as the running body 10 moves. In the present embodiment, as the running body 10 moves, the temperature of the spinnerets 31 of the spinning apparatuses 3 can be measured by the thermal camera 9 provided at the running body 10. It is therefore unnecessary to provide the thermal camera 9 for each spinning apparatus 3 when the spinning apparatuses 3 are aligned in one direction.

The yarn spinning system 1 of the present embodiment further includes the cooler 5 which is provided below each spinning apparatus 3 and which is configured to cool the yarns Y spun out from the spinnerets 31. The reflector 8 is provided below the cooler 5. When the cooler 5 is provided below the spinning apparatus 3, the thermal camera 9 needs to be provided further in the vicinity of the yarn paths of the yarns Y so that the temperature of each spinneret 31 is directly measured by the thermal camera 9. Because of this, the thermal camera 9 is more likely to be damaged by the falling hot molten polymer or to be contaminated by the yarn dust. As a result, a problem in which the measurement of temperature is impossible is also more likely to occur. Therefore, it is further effective to adopt an arrangement in which (i) the reflector 8 which is more resistant to damage and contamination than the thermal camera 9 is provided in the vicinity of the yarn paths of the yarns Y, (ii) a beam of infrared light radiated from each spinneret 31 is reflected by the reflector 8, and (iii) the reflected beam of infrared light is received by the thermal camera 9.

The yarn spinning system 1 of the present embodiment further includes the oil applicator 6 which is provided below the spinning apparatus 3 and which includes the oil supply guides 61 configured to apply oil to the yarns Y spun out from the spinnerets 31. The reflector 8 is attached to the oil applicator 6. It is relatively specious around the oil applicator 6. This facilitates the attachment of the reflector 8.

The yarn spinning system 1 of the present embodiment is structured so that the reflector 8 is able to move up and down, and further includes the angle adjusting mechanism 7 configured to adjust the reflection angle of each beam of the infrared light at the reflector 8. With this arrangement, even when the position of the reflector 8 is changed in the up-down direction, the angle adjusting mechanism 7 adjusts the reflection angle of each beam of the infrared light at the reflector 8 so that a beam of infrared light radiated from each spinneret 31 is reflected toward the thermal camera 9 by the reflector 8.

In the yarn spinning system 1 of the present embodiment, the thermal camera 9 is able to move up and down. This allows the thermal camera 9 to move up and down as the reflector 8 moves up and down. The thermal camera 9 is therefore able to receive each beam of infrared light reflected by the reflector 8, at a suitable position.

The embodiment of the present invention is described hereinabove. However, the specific structure of the present invention shall not be interpreted as to be limited to the above-described embodiment. The scope of the present invention is defined not by the above embodiment but by claims set forth below, and shall encompass the equivalents in the meaning of the claims and every modification within the scope of the claims.

In the embodiment above, the single reflector 8 is provided at a position on which beams of infrared light radiated from all the spinnerets 31 included in each spinning apparatus 3 are incident. However, the disclosure is not limited to this. The position of the reflector 8 may be differently arranged as long as the reflector 8 is provided at a position on which at least one beam of infrared light radiated from at least one spinneret 31 is incident. In order to measure the temperature distribution of a single entire spinneret 31, the reflector 8 is preferably provided at a position on which a beam of infrared light radiated from the single entire spinneret 31 is incident. In order to grasp how uneven the temperature is between the spinnerets 31, the reflector 8 is preferably provided at a position on which beams of infrared light radiated from the spinnerets 31 are incident. Plural reflectors 8 may be provided. The reflectors 8 may be provided to correspond to the respective spinnerets 31.

In the embodiment above, the thermal camera 9 is configured to take an image showing the temperature distribution of each spinneret 31. However, the disclosure is not limited to this. An infrared light sensor, which is able to measure the temperature based on the amount of energy of each received beam of infrared light, may be used as long as the temperature of each spinneret 31 can be measured. For example, a value of the measured temperature of each spinneret 31 may be output.

In the embodiment above, the thermal camera 9 is able to receive beams of infrared light at once which are radiated from the spinnerets 31 and which are reflected by the single reflector 8. However, the disclosure is not limited to this. For example, the thermal camera 9 may be able to receive the beams of infrared light at once which are radiated from the spinnerets 31 and which are reflected by plural reflectors 8. That is, the thermal camera 9 may be able to receive the beams of infrared light reflected by the different reflectors 8 at once. The thermal camera 9 may be configured to receive a beam of infrared light at once which is radiated from only one spinneret 31 and which is reflected by the reflector 8.

In the embodiment above, the thermal camera 9 is able to measure the temperature of each of the spinnerets 31 of the spinning apparatuses 3. However, the disclosure is not limited to this. The thermal camera 9 may be able to measure the temperature of each of the spinnerets 31 of at least one spinning apparatus 3.

In the embodiment above, the spinning apparatuses 3 aligned in the left-right direction are provided, and each spinning apparatus 3 includes the spinnerets 31. However, the disclosure is not limited to this. The number of the spinnerets 31 included in each spinning apparatus 3 may be one. The number of the spinning apparatuses 3 may be one.

In the embodiment above, the thermal camera 9 is attached to the running body 10 configured to run with the tire 10a on the partition floor 2. However, the disclosure is not limited to this. The running body 10 to which the thermal camera 9 is attached may be configured to run along a rail attached to the partition floor 2, a side wall, a ceiling, or the like. The thermal camera 9 may be attached to a flying object such as a drone, etc. The thermal camera 9 may be attached not to such a moving body but to a fixed member which does not move. The thermal camera 9 may be portable by the operator.

In the embodiment above, the cooler 5 is provided below the spinning apparatus 3. However, the disclosure is not limited to this. The present invention may be applied to a system which does not include the cooler 5.

In the embodiment above, the reflector 8 is attached to the cover 62 of the oil applicator 6. However, the layout of the reflector 8 is not limited to this. For example, the reflector 8 may be attached to a member which is different from the cover 62 and which is one of members forming the oil applicator 6. The reflector 8 may be attached to a device or member different from the oil applicator 6. The reflector 8 may be the cover 62 of the oil applicator 6 itself. This reduces the number of members.

In the embodiment above, the angle adjusting mechanism 7 configured to adjust the reflection angle of each beam of the infrared light at the reflector 8 is provided. However, the disclosure is not limited to this. The reflection angle of each beam of the infrared light at the reflector 8 may be a fixed angle.

In the embodiment above, both the reflector 8 and the thermal camera 9 are able to move up and down. However, the disclosure is not limited to this. Only one of the reflector 8 and the thermal camera 9 may be able to move up and down. Both the reflector 8 and the thermal camera 9 may not be movable in the up-down direction.

## Claims

1. A yarn spinning system (1) comprising: at least one spinning apparatus (3) including at least one spinneret (31) configured to spin out a yarn (Y);
at least one reflector (8) which is provided at a position on which at least one beam of infrared light radiated from the at least one spinneret (31) is incident, and which is configured to reflect the at least one incident beam of the infrared light; and
an infrared light sensor (9) which is able to measure the temperature of the at least one spinneret (31) by receiving the at least one beam of the infrared light reflected by the at least one reflector (8), and
the at least one reflector (8) being opposite to the infrared light sensor (9) over a yarn path of the yarn (Y) spun out from the at least one spinneret (31).

2. The yarn spinning system (1) according to claim 1, wherein, the infrared light sensor (9) is able to measure the temperature distribution of the at least one spinneret (31).

3. The yarn spinning system (1) according to claim 1 or 2, wherein, the at least one reflector (8) is able to reflect beams of the infrared light radiated from spinnerets (31).

4. The yarn spinning system (1) according to any one of claims 1 to 3, wherein, the infrared light sensor (9) is able to receive the beams of the infrared light at once which are radiated from the spinnerets (31) and which are reflected by the at least one reflector (8).

5. The yarn spinning system (1) according to any one of claims 1 to 4, further comprising a movable moving body (10), wherein,
the infrared light sensor (9) is provided at the moving body (10), and
as the moving body (10) moves, the infrared light sensor (9) is able to receive the beams of the infrared light which are radiated from the spinnerets (31) and which are reflected by the at least one reflector (8).

6. The yarn spinning system (1) according to claim 5, comprising spinning apparatuses (3) aligned in one direction, wherein,
the moving body (10) is movable across the spinning apparatuses (3), and
as the moving body (10) moves, the infrared light sensor (9) is able to measure the temperature of each of the spinnerets (31) of the spinning apparatuses (3).

7. The yarn spinning system (1) according to any one of claims 1 to 6, further comprising a cooler (5) which is provided below each of the spinning apparatuses (3) and which is configured to cool the yarn (Y) spun out from the each of the spinnerets (31), wherein,
the at least one reflector (8) is provided below the cooler (5).

8. The yarn spinning system (1) according to any one of claims 1 to 7, further comprising an oil applicator (6) which is provided below the each of the spinning apparatuses (3) and which includes an oil supply guide (61) configured to apply oil to the yarn (Y) spun out from the each of the spinnerets (31), wherein,
the at least one reflector (8) is attached to the oil applicator (6).

9. The yarn spinning system (1) according to claim 8, wherein, the oil applicator (6) further includes a cover (62) which is provided above the oil supply guide (61) and which is movable between a position where the cover (62) does not cover the oil supply guide (61) and a position where the cover (62) covers the oil supply guide (61), and
the cover (62) functions as the at least one reflector (8).

10. The yarn spinning system (1) according to any one of claims 1 to 9, wherein, the at least one reflector (8) is able to move up and down, and
an angle adjusting mechanism (7) is provided to adjust a reflection angle of each of the beams of the infrared light at the at least one reflector (8).

11. The yarn spinning system (1) according to claim 10, wherein, the infrared light sensor (9) is able to move up and down.

## Patentansprüche

1. Garnspinnsystem (1), umfassend: mindestens eine Spinneinrichtung (3), die mindestens eine Spinndüse (31) einschließt, die zum Verspinnen eines Garns (Y) konfiguriert ist;
mindestens einen Reflektor (8), der an einer Position bereitgestellt ist, an der mindestens ein von der mindestens einen Spinndüse (31) ausgestrahlter Infrarotlichtstrahl auftrifft, und der so konfiguriert ist, dass er den mindestens einen auftreffenden Infrarotlichtstrahl reflektiert; und
einen Infrarotlichtsensor (9), der in der Lage ist, die Temperatur der mindestens einen Spinndüse (31) zu messen, indem er den mindestens einen Strahl des Infrarotlichts empfängt, der von dem mindestens einen Reflektor (8) reflektiert wird, und
wobei sich der mindestens eine Reflektor (8) gegenüber dem Infrarotlichtsensor (9) über einem Garnpfad des aus der mindestens einen Spinndüse (31) ausgesponnenen Garns (Y) befindet.

2. Garnspinnsystem (1) nach Anspruch 1, wobei der Infrarotlichtsensor (9) in der Lage ist, die Temperaturverteilung der mindestens einen Spinndüse (31) zu messen.

3. Garnspinnsystem (1) nach Anspruch 1 oder 2, wobei der mindestens eine Reflektor (8) in der Lage ist, von Spinndüsen (31) ausgestrahlte Infrarotlichtstrahlen zu reflektieren.

4. Garnspinnsystem (1) nach einem der Ansprüche 1 bis 3, wobei der Infrarotlichtsensor (9) in der Lage ist, die von den Spinndüsen (31) ausgestrahlten und von dem mindestens einen Reflektor (8) reflektierten Infrarotlichtstrahlen gleichzeitig zu empfangen.

5. Garnspinnsystem (1) nach einem der Ansprüche 1 bis 4, weiter umfassend einen beweglichen sich bewegenden Körper (10), wobei,
der Infrarotlichtsensor (9) am sich bewegenden Körper (10) vorgesehen ist, und
wenn sich der sich bewegende Körper (10) bewegt, der Infrarotsensor (9) die Infrarotstrahlen empfangen kann, die von den Spinndüsen (31) ausgestrahlt werden und von dem mindestens einen Reflektor (8) reflektiert werden.

6. Garnspinnsystem (1) nach Anspruch 5, umfassend in einer Richtung ausgerichtete Spinneinrichtungen (3), wobei,
der sich bewegende Körper (10) über die Spinneinrichtungen (3) beweglich ist, und
wenn sich der sich bewegende Körper (10) bewegt, der Infrarotlichtsensor (9) die Temperatur jeder der Spinndüsen (31) der Spinneinrichtungen (3) messen kann.

7. Garnspinnsystem (1) nach einem der Ansprüche 1 bis 6, weiter umfassend einen Kühler (5), der unterhalb jeder der Spinneinrichtungen (3) bereitgestellt ist und der so konfiguriert ist, dass er das aus jeder der Spinndüsen (31) ausgesponnene Garn (Y) kühlt, wobei,
der mindestens eine Reflektor (8) unterhalb des Kühlers (5) bereitgestellt ist.

8. Garnspinnsystem (1) nach einem der Ansprüche 1 bis 7, weiter umfassend einen Ölaufgeber (6), der unterhalb jeder der Spinneinrichtungen (3) bereitgestellt ist und der eine Ölzufuhrführung (61) einschließt, die so konfiguriert ist, dass sie Öl auf das aus jeder der Spinndüsen (31) ausgesponnene Garn (Y) aufträgt, wobei,
der mindestens eine Reflektor (8) an dem Ölaufgeber (6) angebracht ist.

9. Garnspinnsystem (1) nach Anspruch 8, wobei der Ölaufgeber (6) weiter eine Abdeckung (62) einschließt, die über der Ölzufuhrführung (61) bereitgestellt ist und die zwischen einer Position, in der die Abdeckung (62) die Ölzufuhrführung (61) nicht abdeckt, und einer Position, in der die Abdeckung (62) die Ölzufuhrführung (61) abdeckt, bewegbar ist, und
wobei die Abdeckung (62) als der mindestens eine Reflektor (8) fungiert.

10. Garnspinnsystem (1) nach einem der Ansprüche 1 bis 9, wobei der mindestens eine Reflektor (8) in der Lage ist, sich auf und ab zu bewegen, und
ein Winkelverstellmechanismus (7) bereitgestellt ist, um den Reflexionswinkel jedes der Infrarotlichtstrahlen an dem mindestens einen Reflektor (8) einzustellen.

11. Garnspinnsystem (1) nach Anspruch 10, wobei der Infrarotlichtsensor (9) in der Lage ist, sich auf und ab zu bewegen.

## Revendications

1. Système (1) de filage de fil comprenant : au moins un appareil de filage (3) incluant au moins une filière (31) configurée pour filer un fil (Y) ;
au moins un réflecteur (8) qui est prévu à une position sur laquelle est incident au moins un faisceau de rayonnement infrarouge émis par l'au moins une filière (31), et qui est configuré pour réfléchir l'au moins un faisceau incident de rayonnement infrarouge ; et
un capteur (9) de rayonnement infrarouge qui est conçu pour mesurer la température de l'au moins une filière (31) en recevant l'au moins un faisceau de rayonnement infrarouge réfléchi par l'au moins un réflecteur (8), et
l'au moins un réflecteur (8) étant opposé au capteur (9) de rayonnement infrarouge sur un chemin de fil du fil (Y) filé à partir de l'au moins une filière (31).

2. Système (1) de filage de fil selon la revendication 1, dans lequel, le capteur (9) de rayonnement infrarouge est conçu pour mesurer la distribution de température de l'au moins une filière (31).

3. Système (1) de filage de fil selon la revendication 1 ou la revendication 2, dans lequel, l'au moins un réflecteur (8) est conçu pour réfléchir des faisceaux de rayonnement infrarouge émis par des filières (31).

4. Système (1) de filage de fil selon l'une quelconque des revendications 1 à 3, dans lequel, le capteur (9) de rayonnement infrarouge est conçu pour recevoir à la fois les faisceaux de rayonnement infrarouge qui sont émis par les filières (31) et qui sont réfléchis par l'au moins un réflecteur (8).

5. Système (1) de filage de fil selon l'une quelconque des revendications 1 à 4, comprenant en outre un corps mobile (10) déplaçable, dans lequel,
le corps mobile (10) est muni du capteur (9) de rayonnement infrarouge, et
à mesure que le corps mobile (10) se déplace, le capteur (9) de rayonnement infrarouge est conçu pour recevoir les faisceaux de rayonnement infrarouge qui sont émis par les filières (31) et qui sont réfléchis par l'au moins un réflecteur (8).

6. Système (1) de filage de fil selon la revendication 5, comprenant des appareils de filage (3) alignés dans une direction, dans lequel,
le corps mobile (10) est déplaçable à travers les appareils de filage (3), et
à mesure que le corps mobile (10) se déplace, le capteur (9) de rayonnement infrarouge est conçu pour mesurer la température de chacune des filières (31) des appareils de filage (3).

7. Système (1) de filage de fil selon l'une quelconque des revendications 1 à 6, comprenant en outre un refroidisseur (5) qui est prévu sous chacun des appareils de filage (3) et qui est configuré pour refroidir le fil (Y) filé à partir de chacune des filières (31), dans lequel,
l'au moins un réflecteur (8) est prévu sous le refroidisseur (5).

8. Système (1) de filage de fil selon l'une quelconque des revendications 1 à 7, comprenant en outre un applicateur d'huile (6) qui est prévu sous chacun des appareils de filage (3) et qui inclut un guide (61) d'alimentation en huile configuré pour appliquer de l'huile au fil (Y) filé à partir de chacune des filières (31), dans lequel,
l'au moins un réflecteur (8) est fixé à l'applicateur d'huile (6).

9. Système (1) de filage de fil selon la revendication 8, dans lequel, l'applicateur d'huile (6) inclut en outre un couvercle (62) qui est prévu au-dessus du guide (61) d'alimentation en huile et qui est déplaçable entre une position où le couvercle (62) ne couvre pas le guide (61) d'alimentation en huile et une position où le couvercle (62) couvre le guide (61) d'alimentation en huile, et le couvercle (62) fonctionne comme l'au moins un réflecteur (8).

10. Système (1) de filage de fil selon l'une quelconque des revendications 1 à 9, dans lequel, l'au moins un réflecteur (8) est conçu pour monter et descendre, et
un mécanisme (7) de réglage d'angle est prévu pour régler un angle de réflexion de chacun des faisceaux de rayonnement infrarouge au niveau de l'au moins un réflecteur (8).

11. Système (1) de filage de fil selon la revendication 10, dans lequel, le capteur (9) de rayonnement infrarouge est conçu pour monter et descendre.
